# EUROPEAN PATENT APPLICATION

(11) **EP 0 783 887 A2**
(43) Date of publication of application: **16.07.1997**
(21) Application number: 96309403.2
(22) Date of filing: 23.12.1996
(51) Int. Cl.: A61K 31/41, A61K 47/38

(54) **Topical compositions for the treatment of hyperproliferative skin disease**

(30) Priority: 11.01.1996 GB 9600545
(71) Applicant: CIBA-GEIGY AG, 4002 Basel (CH)
(72) Inventor: Taylor, Peter William, Billinghurst, West Sussex, RH14 9HF (GB); Wright, Judith Melanie, Horsham, West Sussex, RH12 1SE (GB)
(74) Representative: Sharman, Thomas

(57) **Abstract**

A composition suitable for topical administration in treatment of a hyperproliferative skin disease which is a gel comprising (A) a compound of formula 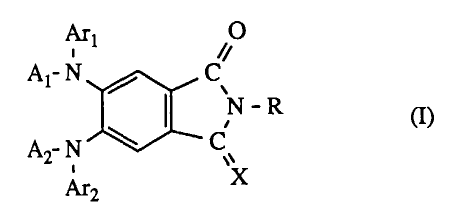 wherein A₁ and A₂ are each independently of the other hydrogen, lower alkyl, lower alkenyl, lower alkynyl, aryl, acyl, lower alkylsulfonyl or arylsulfonyl; or wherein A₁ and A₂ together form unsubstituted or lower alkyl- or hydroxy-substituted lower alkylene; Ar₁ and Ar₂ are each independently of the other aryl, heteroaryl or unsubstituted or substituted cycloalkyl; the group -C(=X)- is -C(=O)-, -C(=S)-, -CH₂- or -C(=CR₁R₂)- wherein R₁ and R₂ are each independently of the other hydrogen or lower alkyl; and R is hydrogen, lower alkyl, aryl-lower alkyl, aryl, amino, hydroxy or lower alkoxy; with the proviso that R is other than phenyl when A₁ and A₂ are hydrogen, Ar₁ and Ar₂ are phenyl and the group -C(=X)- is -C(=O)-; or a pharmaceutically acceptable salt thereof when a salt-forming group is present; (B)a C₁-C₄ alkyl monoether of diethyleneglycol, (C) a gelling agent and (D) a solvent which is miscible with (B).

## Description

This invention relates to compositions which are suitable for topical administration in the treatment of hyperproliferative skin diseases such as psoriasis.

In EP 0516588, there are described substituted diaminophthalimides and analogues thereof which can be used as active ingredients, inter alia, in the treatment of tumours and psoriasis. Various components for orally and parenterally administrable compositions containing the active ingredients are described.

The formulation of a topically administrable composition containing an active ingredient of EP 0516588, particularly for use in the treatment of hyperproliferative skin diseases such as psoriasis, has proved problematic because, inter alia, components conventionally used in the formulation of topically administrable pharmaceutical compositions have an adverse effect on stability of the active ingredient and compositions containing conventionally used carriers show poor uptake of the active ingredient into the epidermis and dermis of human skin.

It has now been found that by incorporating an active ingredient such as described in EP 0516588 in a specially selected carrier system, a stable topically adminstrable composition can be produced which exhibits sufficient uptake of the active ingredient by the epidermis and dermis of human skin, even at low dosages of the active ingredient, for it to be used in the treatment of hyperproliferative skin diseases, particularly psoriasis.

Accordingly, the present invention provides, in one aspect, a composition suitable for topical administration in the treatment of hyperproliferative skin diseases which is a gel comprising (A) a compound of formula 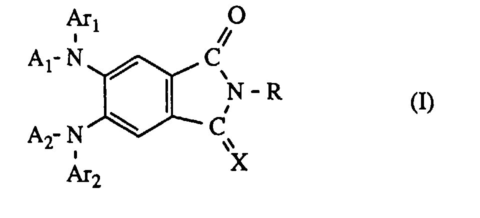 wherein A₁ and A₂ are each independently of the other hydrogen, lower alkyl, lower alkenyl, lower alkynyl, aryl, acyl, lower alkylsulfonyl or arylsulfonyl; or wherein A₁ and A₂ together form unsubstituted or lower alkyl- or hydroxy-substituted lower alkylene; Ar₁ and Ar₂ are each independently of the other aryl, heteroaryl or unsubstituted or substituted cycloalkyl; the group -C(=X)- is -C(=O)-, -C(=S)-, -CH₂- or -C(=CR₁R₂)- wherein R₁ and R₂ are each independently of the other hydrogen or lower alkyl; and R is hydrogen, lower alkyl, aryl-lower alkyl, aryl, amino, hydroxy or lower alkoxy; with the proviso that R is other than phenyl when A₁ and A₂ are hydrogen, Ar₁ and Ar₂ are phenyl and the group -C(=X)- is -C(=O)-; or a pharmaceutically acceptable salt thereof when a salt-forming group is present; (B) a C₁-C₄ alkyl monoether of diethyleneglycol, (C) a gelling agent and (D) a solvent which is miscible with (B).

The general terms used hereinbefore and hereinafter preferably have the following meanings within the scope of this specification:

The term "lower" denotes a radical having up to and including 7, especially up to and including 4, and more especially having 1 or 2, carbon atoms.

Lower alkyl is preferably n-propyl, isopropyl, n-butyl, isobutyl, sec-butyl, tert-butyl, n-pentyl, neopentyl, n-hexyl or n-heptyl, preferably ethyl and especially methyl.

Lower alkenyl has from 2 to 7, preferably from 2 to 4, carbon atoms and is, for example, allyl or crotyl.

Lower alkynyl has from 2 to 7, preferably from 2 to 4, carbon atoms and is, for example, propyn-1-yl or propyn-2-yl or 2-butyn-1-yl.

Lower alkyl substituted by halogen is, for example, trifluoromethyl.

Aryl is preferably phenyl or naphthyl, such as 1- or 2-naphthyl. The phenyl and naphthyl radicals can be unsubstituted or substituted, especially as indicated below for phenyl. Aryl is preferably phenyl that is unsubstituted or substituted by one or more, especially one to three, for example one or two, substituents selected independently from the group consisting of: hydrocarbyl, for example lower alkyl, lower alkenyl, lower alkynyl, lower alkylene (linked to two adjacent carbon atoms), cycloalkyl, phenyl-lower alkyl or phenyl; substituted hydrocarbyl, for example lower alkyl substituted, for example, by hydroxy, lower alkoxy, phenyl-lower alkoxy, lower alkanoyloxy, halogen, amino, lower alkylamino, di-lower alkylamino, mercapto, lower alkylthio, lower alkylsulfinyl, lower alkylsulfonyl, carboxy, lower alkoxycarbonyl, carbamoyl, N-lower alkylcarbamoyl, N,N-di-lower alkylcarbamoyl and/or by cyano; hydroxy; etherified hydroxy, for example lower alkoxy, halo-lower alkoxy, phenyl-lower alkoxy, phenoxy, lower alkenyloxy, halo-lower alkenyloxy, lower alkynyloxy or lower alkylenedioxy (linked to two adjacent carbon atoms); esterified hydroxy, for example lower alkanoyloxy, phenyl-lower alkanoyloxy or phenylcarbonyloxy (≙ benzoyloxy); mercapto; etherified mercapto, which is optionally oxidised, for example lower alkylthio, phenyl-lower alkylthio, phenylthio, lower alkylsulfinyl [-S(=O)-lower alkyl], phenyl-lower alkylsulfinyl, phenylsulfinyl, lower alkylsulfonyl [-S(O₂)-lower alkyl], phenyl-lower alkylsulfonyl or phenylsulfonyl; halogen; nitro; amino; monohydrocarbylamino, for example lower alkylamino, cycloalkylamino, phenyl-lower alkylamino or phenylamino; dihydrocarbylamino, for example di-lower alkylamino, N-lower alkyl-N-phenylamino, N-lower alkyl-N-phenyl-lower alkylamino, lower alkyleneamino, or lower alkyleneamino interrupted by -O-, -S- or -NR'' (wherein R'' is hydrogen, alkyl or acyl); acylamino, for example lower alkanoylamino, phenyl-lower alkanoylamino or phenylcarbonylamino (≙ benzoylamino); acyl, for example lower alkanoyl, phenyl-lower alkanoyl or phenylcarbonyl (= benzoyl); carboxy; esterified carboxy, for example lower alkoxycarbonyl; amidated carboxy, for example carbamoyl, N-lower alkylcarbamoyl, N,N-di-lower alkylcarbamoyl, N-hydroxycarbamoyl or N-phenylcarbamoyl; cyano; sulfo (SO₃H); esterified sulfo, for example lower alkoxysulfonyl; and amidated sulfo, for example sulfamoyl (SO₂NH₂), N-lower alkylsulfamoyl, N,N-di-lower alkylsulfamoyl or N-phenylsulfamoyl; phenyl groups occurring in the substituents each being unsubstituted or substituted by lower alkyl, lower alkoxy, hydroxy, halogen and/or by trifluoromethyl.

R is preferably selected from all the above-mentioned definitions except aryl.

Heteroaryl is a heterocyclic radical of aromatic character and is preferably linked via a ring carbon atom. It is especially a 5- or 6-membered ring, for example imidazolyl, triazolyl, pyridyl, pyrimidinyl or triazinyl, and especially pyridyl. Those radicals may be unsubstituted or substituted, for example, by lower alkyl, hydroxy, lower alkoxy, halogen, cyano and/or by trifluoromethyl.

Pyridyl is, for example, 2-,3- or 4-pyridyl.

Imidazolyl is, for example, 2- or 4(5)-imidazolyl.

Triazolyl is, for example, 1,2,4-triazol-3- or -4-yl or 1,2,3-triazol-4-yl.

Pyrimidinyl is, for example, 2-, 4- or 5-pyrimidinyl.

Triazinyl is, for example, 1,3,5-triazin-2-yl.

Lower alkylene, formed from A₁ and A₂ together, is unbranched and is especially a (CH₂)ₙ group wherein n is from 1 to 4, preferably 2 or 3. It is preferably unsubstituted but may also be substituted by lower alkyl or hydroxy.

Lower alkylene, linked to two adjacent carbon atoms of a benzene ring, is preferably C₃-C₄alkylene, for example 1,3-propylene or 1,4-butylene.

Lower alkylenedioxy, linked to two adjacent carbon atoms, is preferably C₁-C₂alkylenedioxy, for example methylene- or 1,2-ethylene-dioxy.

Lower alkyleneamino is preferably C₄-C₇- and especially C₄-C₅-alkyleneamino, for example piperidino. Lower alkyleneamino interrupted by -O-, -S- or -NR''- is preferably C₄-C₇- and especially C₄-C₅-alkyleneamino in which a ring carbon atom has been replaced by the corresponding hetero group, and is especially morpholino, thiomorpholino, piperazino or 4-lower alkyl- or 4-lower alkanoyl-piperazino.

Acyl is preferably lower alkanoyl, halo-lower alkanoyl, aryl-lower alkanoyl or arylcarbonyl. Acyl is especially lower alkanoyl.

Lower alkanoyl is preferably formyl, acetyl, propionyl, n-butyryl, pivaloyl or valeroyl, especially acetyl.

Aryl-lower alkyl is preferably phenyl-lower alkyl and especially benzyl.

Cycloalkyl is preferably C₃-C₈- and especially C₅-C₇-cycloalkyl, which is intended to indicate that it contains from 3 to 8 and from 5 to 7 ring carbon atoms, respectively. It may, however, also be substituted, for example by lower alkyl or hydroxy.

Halogen is especially fluorine, chlorine and bromine, but may also be iodine.

Salts of compounds of formula I having salt-forming groups are especially pharmaceutically acceptable, non-toxic salts. For example, compounds of formula I having basic groups may form acid addition salts, for example with inorganic acids, such as hydrochloric acid, sulfuric acid or phosphoric acid, or with suitable organic carboxylic or sulfonic acids, for example acetic acid, fumaric acid or methanesulfonic acid, or with amino acids, such as arginine or lysine. Compounds of formula I having an acidic group, for example carboxy, form, for example, metal or ammonium salts, such as alkali metal and alkaline earth metal salts, for example sodium, potassium, magnesium or calcium salts, and also ammonium salts with ammonia or suitable organic amines, such as lower alkylamines, for example triethylamine, hydroxy-lower alkylamines, for example 2-hydroxyethylamine, bis(2-hydroxyethyl)amine or tris(2-hydroxyethyl)amine, basic aliphatic esters of carboxylic acids, for example 4-aminobenzoic acid 2-diethylaminoethyl ester, lower alkyleneamines, for example 1-ethylpiperidine, cycloalkylamines, for example dicyclohexylamine, or benzylamines, for example N,N'-dibenzylethylenediamine, dibenzylamine or benzyl-β-phenethylamine. Compounds of formula I having an acidic group and a basic group can also be in the form of internal salts, that is to say in zwitterionic form.

The salts of compounds of formula I also include complexes of compounds of formula I (A₁, A₂ = hydrogen) with transition metal ions, for example copper, cobalt or manganese.

Preferred compounds of formula I are those wherein A₁ and A₂ are each independently of the other hydrogen, lower alkyl, aryl, acyl, lower alkylsulfonyl or arylsulfonyl; or wherein A₁ and A₂ together are unsubstituted or lower alkyl- or hydroxy-substituted lower alkylene; Ar₁ and Ar₂ are each independently of the other aryl, heteroaryl or unsubstituted or substituted cycloalkyl; the group -C(=X)- is -C(=O)-, -C(=S)-, -CH₂- or -C(=CR₁R₂)-wherein R₁ and R₂ are each independently of the other hydrogen or lower alkyl; and R is hydrogen, lower alkyl, aryl-lower alkyl, aryl, amino, hydroxy or lower alkoxy; with the proviso that R is other than phenyl when A₁ and A₂ are hydrogen, Ar₁ and Ar₂ are phenyl and the group -C(=X)- is -C(=O)-, and pharmaceutically acceptable salts thereof when salt-forming groups are present.

More preferred compounds of formula I are those wherein A₁ and A₂ are each independently of the other hydrogen, lower alkyl; lower alkenyl; phenyl or 1-naphthyl or 2-naphthyl, the three last-mentioned radicals being unsubstituted or substituted by lower alkyl, lower alkoxy, hydroxy, halogen and/or by trifluoromethyl; lower alkanoyl, lower alkylsulfonyl or phenylsulfonyl wherein the phenyl group is unsubstituted or substituted by lower alkyl, lower alkoxy, hydroxy, halogen and/or by trifluoromethyl; or wherein A₁ and A₂ together are C₁-C₄alkylene; wherein Ar₁ and Ar₂ are each independently of the other phenyl or naphthyl, each of which is unsubstituted or substituted by one or more substituents from the group consisting of: lower alkyl, lower alkenyl, lower alkynyl, lower alkylene (linked to two adjacent carbon atoms), C₃-C₈cycloalkyl, phenyl-lower alkyl, phenyl; lower alkyl substituted by hydroxy, lower alkoxy, phenyl-lower alkoxy, lower alkanoyloxy, halogen, amino, lower alkylamino, di-lower alkylamino, mercapto, lower alkylthio, lower alkylsulfinyl, lower alkylsulfonyl, carboxy, lower alkoxycarbonyl, carbamoyl, N-lower alkylcarbamoyl, N,N-di-lower alkylcarbamoyl and/or by cyano; hydroxy, lower alkoxy, halo-lower alkoxy, phenyl-lower alkoxy, phenoxy, lower alkenyloxy, halo-lower alkenyloxy, lower alkynyloxy, lower alkylenedioxy (linked to two adjacent carbon atoms), lower alkanoyloxy, phenyl-lower alkanoyloxy, phenylcarbonyloxy, mercapto, lower alkylthio, phenyl-lower alkylthio, phenylthio, lower alkylsulfinyl, phenyl-lower alkylsulfinyl, phenylsulfinyl, lower alkylsulfonyl, phenylalkylsulfonyl, phenylsulfonyl, halogen, nitro, amino, lower alkylamino, C₃-C₈cycloalkylamino, phenyl-lower alkylamino, phenylamino, di-lower alkylamino, N-lower alkyl-N-phenylamino, N-lower alkyl-N-phenyl-lower alkylamino, lower alkyleneamino, lower alkyleneamino interrupted by -O-, -S- or -NR'' (wherein R'' is hydrogen, lower alkyl or lower alkanoyl), lower alkanoylamino, phenyl-lower alkanoylamino, phenylcarbonylamino, lower alkanoyl, phenyl-lower alkanoyl, phenylcarbonyl, carboxy, lower alkoxycarbonyl, carbamoyl, N-lower alkylcarbamoyl, N,N-di-lower alkylcarbamoyl, N-hydroxycarbamoyl, N-phenylcarbamoyl, cyano, sulfo, lower alkylsulfonyl, sulfamoyl, N-lower alkylsulfamoyl, N,N-di-lower alkylsulfamoyl and N-phenylsulfamoyl (phenyl groups occurring in the substituents each being unsubstituted or substituted by lower alkyl, lower alkoxy, hydroxy, halogen and/or by trifluoromethyl); pyridyl or pyrimidinyl that is unsubstituted or substituted by lower alkyl, hydroxy, lower alkoxy, halogen, cyano and/or by trifluoromethyl; or C₃-C₈cycloalkyl; the group -C(=X)- is -C(=O)-, -C(=S)-, -CH₂- or -C(=CR₁R₂)- wherein R₁ and R₂ are each independently of the other hydrogen or lower alkyl, and R is hydrogen, lower alkyl, phenyl-lower alkyl, phenyl, 1-naphthyl or 2-naphthyl, in the four last-mentioned radicals the phenyl or naphthyl group being unsubstituted or substituted by lower alkyl, lower alkoxy, hydroxy, halogen and/or by trifluoromethyl; or R is amino, hydroxy or lower alkoxy, with the proviso that R is other than phenyl when A₁ and A₂ are hydrogen, Ar₁ and Ar₂ are phenyl and the group -C(=X)- is -C(=O)-; and pharmaceutically acceptable salts thereof when salt-forming groups are present.

Of those last-mentioned compounds of formula I, special preference is given to those wherein R is as defined except for phenyl, and the other radicals are as defined, and salts thereof when salt-forming groups are present.

Further preferred compounds of formula I are those wherein A₁ and A₂ are each independently of the other hydrogen, lower alkyl; phenyl or 1-naphthyl or 2-naphthyl, the three last-mentioned radicals being unsubstituted or substituted by lower alkyl, lower alkoxy, hydroxy, halogen and/or by trifluoromethyl; lower alkanoyl, lower alkylsulfonyl or phenylsulfonyl wherein the phenyl group is unsubstituted or substituted by lower alkyl, lower alkoxy, hydroxy, halogen and/or by trifluoromethyl; or wherein A₁ and A₂ together are C₁-C₄alkylene; wherein Ar₁ and Ar₂ are each independently of the other phenyl or naphthyl, each of which is unsubstituted or substituted by one or more substituents from the group consisting of: lower alkyl, lower alkenyl, lower alkynyl, lower alkylene (linked to two adjacent carbon atoms), C₃-C₈cycloalkyl, phenyl-lower alkyl, phenyl; lower alkyl substituted by hydroxy, lower alkoxy, phenyl-lower alkoxy, lower alkanoyloxy, halogen, amino, lower alkylamino, di-lower alkylamino, mercapto, lower alkylthio, lower alkylsulfinyl, lower alkylsulfonyl, carboxy, lower alkoxycarbonyl, carbamoyl, N-lower alkylcarbamoyl, N,N-di-lower alkylcarbamoyl and/or by cyano; hydroxy, lower alkoxy, halo-lower alkoxy, phenyl-lower alkoxy, phenoxy, lower alkenyloxy, halo-lower alkenyloxy, lower alkynyloxy, lower alkylenedioxy (linked to two adjacent carbon atoms), lower alkanoyloxy, phenyl-lower alkanoyloxy, phenylcarbonyloxy, mercapto, lower alkylthio, phenyl-lower alkylthio, phenylthio, lower alkylsulfinyl, phenyl-lower alkylsulfinyl, phenylsulfinyl, lower alkylsulfonyl, phenylalkylsulfonyl, phenylsulfonyl, halogen, nitro, amino, lower alkylamino, C₃-C₈cycloalkylamino, phenyl-lower alkylamino, phenylamino, di-lower alkylamino, N-lower alkyl-N-phenylamino, N-lower alkyl-N-phenyl-lower alkylamino, lower alkyleneamino, lower alkyleneamino interrupted by -O-, -S- or -NR'' (wherein R'' is hydrogen, lower alkyl or lower alkanoyl), lower alkanoylamino, phenyl-lower alkanoylamino, phenylcarbonylamino, lower alkanoyl, phenyl-lower alkanoyl, phenylcarbonyl, carboxy, lower alkoxycarbonyl, carbamoyl, N-lower alkylcarbamoyl, N,N-di-lower alkylcarbamoyl, N-hydroxycarbamoyl, N-phenylcarbamoyl, cyano, sulfo, lower alkylsulfonyl, sulfamoyl, N-lower alkylsulfamoyl, N,N-di-lower alkylsulfamoyl and N-phenylsulfamoyl (phenyl groups occurring in the substituents each being unsubstituted or substituted by lower alkyl, lower alkoxy, hydroxy, halogen and/or by trifluoromethyl); pyridyl that is unsubstituted or substituted by lower alkyl, hydroxy, lower alkoxy, halogen, cyano and/or by trifluoromethyl; or C₃-C₈cycloalkyl; the group -C(=X)- is -C(=O)-, -C(=S)-, -CH₂- or -C(=CR₁R₂)- wherein R₁ and R₂ are each independently of the other hydrogen or lower alkyl, and R is hydrogen, lower alkyl; phenyl-lower alkyl, phenyl, 1-naphthyl or 2-naphthyl, in the four last-mentioned radicals the phenyl or naphthyl group being unsubstituted or substituted by lower alkyl, lower alkoxy, hydroxy, halogen and/or by trifluoromethyl; or R is amino, hydroxy or lower alkoxy, with the proviso that R is other than phenyl when A₁ and A₂ are hydrogen, Ar₁ and Ar₂ are phenyl and the group -C(=X)- is -C(=O)-; and pharmaceutically acceptable salts thereof when salt-forming groups are present.

Among compounds of formula I mentioned in the preceding paragraph, special preference is given to those wherein A₁ and A₂ are each independently of the other hydrogen or lower alkyl, the group -C(=X)- is -C(=O)-, -C(=S)- or -C(=CH₂)-, and R is hydrogen or lower alkyl, and salts thereof.

Even more preferred compounds of formula I are those wherein A₁ and A₂ are each independently of the other hydrogen; lower alkyl; lower alkenyl; or lower alkanoyl; or wherein A₁ and A₂ together are C₁-C₄alkylene; wherein Ar₁ and Ar₂ are each independently of the other phenyl or naphthyl, each of which is unsubstituted or substituted by one or more substituents from the group consisting of: lower alkyl, lower alkylene (linked to two adjacent carbon atoms), hydroxy, phenoxy, halogen, nitro, amino, lower alkylamino, di-lower alkylamino, lower alkanoylamino, carboxy, lower alkoxycarbonyl, carbamoyl, N-lower alkylcarbamoyl, N,N-di-lower alkylcarbamoyl and cyano, or in addition from lower alkoxy; pyridyl; pyrimidinyl; or C₃-C₈cycloalkyl; the group -C(=X)- is -C(=O)- or -C(=S)-, and R is hydrogen, lower alkyl, phenyl-lower alkyl, amino or hydroxy; and pharmaceutically acceptable salts thereof when salt-forming groups are present.

Especially preferred compounds of formula I are those wherein A₁ and A₂ are each independently of the other hydrogen or methyl; or wherein A₁ and A₂ together are -(CH₂)₂- or -(CH₂)₃-; Ar₁ and Ar₂ are each independently of the other phenyl or naphthyl, each of which is unsubstituted or substituted by one or more substituents from the group consisting of: lower alkyl, lower alkoxy, phenyl-lower alkoxy, hydroxy, lower alkanoyloxy, nitro, amino, lower alkylamino, di-lower alkylamino, lower alkanoylamino, halogen, trifluoromethyl, carboxy, lower alkoxycarbonyl, carbamoyl, N-lower alkylcarbamoyl, N,N-di-lower alkylcarbamoyl, cyano, lower alkanoyl, benzoyl, lower alkylsulfonyl and sulfamoyl, N-lower alkylsulfamoyl and N,N-di-lower alkylsulfamoyl; cyclopentyl; cyclohexyl; or pyridyl; the group -C(=X)- is -C(=O)-, -C(=S)- or -C(=CH₂)-, and R is hydrogen or lower alkyl; and pharmaceutically acceptable salts thereof.

More especially preferred compounds of formula I are those wherein A₁ and A₂ are hydrogen; Ar₁ and Ar₂ are each independently of the other phenyl that is unsubstituted or substituted by lower alkyl, trifluoromethyl, phenyl, hydroxy, lower alkoxy, benzyloxy, amino, di-lower alkylamino, lower alkanoylamino, halogen, carboxy, lower alkoxycarbonyl, carbamoyl, N,N-di-lower alkylcarbamoyl or by cyano; or cyclohexyl; the group -C(=X)- is -C(=O)-, -C(=S)- or -C(=CH₂)-, and R is hydrogen; and pharmaceutically acceptable salts thereof.

Most especially preferred compounds of formula I are those wherein A₁ and A₂ are each hydrogen; Ar₁ and Ar₂ are each independently of the other unsubstituted phenyl or phenyl that is substituted by halogen, especially by fluorine; the group -C(=X)- is -C(=O)-, and R is hydrogen; and pharmaceutically acceptable salts thereof. Specific most especially preferred compounds are 4,5-bis (anilino)phthalimide and 4,5-bis(4-fluoroanilino)phthalimide.

In a composition of the invention, the C₁-C₄ alkyl monoether of diethyleneglycol may be a methyl, ethyl, propyl or butyl ether of diethyleneglycol. The methyl and, especially, the ethyl ether are preferred.

The gelling agent (C) may comprise one or more gel-forming components used conventionally in the preparation of pharmaceutical gels for topical administration. The compatibility of the gelling agent with the other components of the composition of the invention can be readily tested in a conventional manner by preparing a gel-forming mixture with the solvent (D) and determining whether the other components can be incorporated in the mixture without phase separation.

The gelling agent may be a gel-forming natural or synthetic polymeric material. Examples of polymeric materials conventionally used as gelling agents are gelatin, starch, cellulosic polymers such as cellulose ethers, for example alkylcelluloses such as methylcellulose and ethylcellulose, hydroxyalkyl celluloses such as hydroxyethylcellulose and hydroxypropylcellulose, and hydroxyalkyl alkylcelluloses such as hydroxyethyl methycellulose, hydroxypropylmethylcellulose and hydroxybutyl methycellulose, cellulose esters such as cellulose acetate phthalate, high molecular weight polyethylene oxides, polyoxyethylene-polyoxypropylene block copolymers, polyoxyethylene-polyoxybutylene block copolymers and carboxyl-containing vinyl polymers, for example homopolymers and copolymers of acrylic acid or methacrylic acid which may be crosslinked with an ethylenically unsaturated monomer such as an allyl ether of pentaerythritol or sucrose.

Particularly good results can be obtained using as the gelling agent, a cellulosic polymer, preferably a cellulose ether, more preferably a hydroxyalkyl cellulose or a hydroxyalkyl alkylcellulose, especially hydroxypropyl cellulose or hydroxypropyl methylcellulose.

The solvent (D) may be, for example, water, an organic solvent such as propylene glycol, a polyethylene glycol, a polypropylene glycol or a mixture of two or more thereof, or a mixture of water with a water-miscible organic solvent such as a polyethylene glycol. In especially preferred embodiments, (D) is water or a polyethylene glycol having a molecular weight up to 650, preferably up to 450, especially 350 to 450.

Other optional excipients which may be incorporated in a composition of the invention include antioxidants such as ascorbyl palmitate or an etherified phenol such as butylated hydroxyanisole (a commerical mixture of 2-tert-butyl-4-methoxyphenol and 3-tert-butyl-4-methoxyphenol) or butylated p-cresol (2,6-di-tert-butyl-p-cresol), and preservatives such as ethanol or an ester of p-hydroxybenzoic acid.

The composition of the invention may suitably contain from 0.005 to 0.5%, e.g. 0.005 to 0.2%, more usually from 0.005 to 0.1%, preferably 0.01 to 0.05%, by weight, of the active ingredient (A). The glycol ether (B) may suitably be present in an amount of 20 to 80%, preferably 40 to 60%, especially 45 to 55%, by weight of the composition. The gelling agent (C) may suitably be present in an amount of 0.5 to 10%, preferably 1 to 5%, by weight of the composition.

A composition of the invention may conveniently be prepared by dissolving the active ingredient (A) in the glycol ether (B), mixing the resulting solution with the solvent (D) and adding the gelling agent (C) to the resulting mixture, usually at ambient temperature. Alternatively, when the solvent is organic, the glycol ether and solvent may be mixed, the active ingredient dissolved in this mixture and the gelling agent added to the resulting solution. In another alternative, a solution of (A) in (B) is mixed with a gel-forming mixture of the gelling agent (C) and the solvent (D), the temperature of the mixture being controlled so that the viscosity of the mixture is such that (A) and (B) are dissolved therein before the gel is set.

A composition of the invention may be used in the treatment of hyperproliferative skin diseases, particularly psoriasis. The composition may be topically applied in a conventional manner to an affected area of a patient's skin to provide thereon a therapeutically effective amount of the active ingredient (A). If desired, the skin may be washed prior to treatment to remove psoriatic scales. The composition is usually applied to affected skin daily, although frequency of application may be modified depending on the severity of the condition to be treated. Treatment can be carried out over an extended period. Accordingly, the invention includes a method of treating a hyperproliferative skin disease, particularly psoriasis, which comprises applying a composition of the invention as hereinbefore described topically to affected skin.

The invention is illustrated by the following Examples, in which parts are by weight unless stated otherwise.

### Example 1

Purified water (50 parts) is heated to 70°C. Hydroxypropyl methylcellulose (3 parts) is added to the heated water whilst stirring vigorously. While maintaining vigorous stirring, the mixture is allowed to cool to 45°C, when the viscosity of the mixture has noticeably increased and the stirring rate is reduced. The mixture is cooled further by means of an iced water jacket and maintained at 10-15°C for 5 minutes, after which it is a thick opaque gel. This gel is heated to 65-70°C, with stirring, and a solution of 4,5-bis(anilino)phthalimide (0.01 part) and ascorbyl palmitate antioxidant (0.03 part) in diethyleneglycol monoethyl ether (50 parts) is added to the heated gel. The mixture is stirred vigorously for 5 minutes at 60-65°C, and then allowed to cool, firstly with stirring to 40°C, and then to ambient temperature to give a gel suitable for topical application in the treatment of psoriasis.

The uptake of the active ingredient from the gel into human skin is measured using a Franz diffusion cell. Human skin obtained from elective abdominoplastic surgery, from which adipose tissue has been removed, is tape stripped 15 times with adhesive tape and dermatomed to a thickness of 500µm to simulate the condition of psoriatic skin. The treated skin is cut into sample pieces which are mounted in Franz diffusion cells (1 cm²) at 37°C for 2 hours, the donor compartment of the cells being loaded with 0.3ml of the gel. The receptor compartment in contact with the dermis contains 50% by volume of human plasma in phosphate buffered saline, pH 7.4. The skin samples are then removed, washed clean with water and placed in a plastic bag, which is submerged in water at 60°C for 10 seconds. The epidermis is scraped gently from the dermis of each of the resulting samples using a scalpel. Each epidermal sample is placed in a vial to which 0.5ml acetonitrile is added. Each dermal sample is then chopped finely and placed in a vial to which 0.5ml acetonitrile is added. The vials containing the dermal and epidermal samples are shaken for 48 hours to dissolve 4,5-bis(anilino)phthalimide contained in the samples in the acetonitrile. The resulting solutions are subjected to HPLC (high performance liquid chromatography) to determine their 4,5-bis(anilino)phthalimide contents and thereby the concentration of the phthalimide in the sample epidermis and dermis. The concentration of the phthalimide in the epidermis is 0.086µg per cm² (average of 5 samples) and the concentration of the phthalimide in the dermis is 0.13µg per cm² (average of 5 samples).

### Example 2

Example 1 is repeated using 0.02 part of the bis(anilino)phthalimide instead of the amount used in Example 1, to obtain a gel suitable for topical application in the treatment of psoriasis. The uptake of the phthalimide from the gel into human skin is tested as in Example 1. The concentration of the phthalimide in the epidermis is 1.23µg per cm² and the concentration of the phthalimide in the dermis is 1.40µg per cm² (average of 7 samples in each case).

### Example 3

Example 1 is repeated using 0.03 part of the bis(anilino)phthalimide instead of the amount used in Example 1, to obtain a gel suitable for topical application in the treatment of psoriasis. The uptake of the phthalimide from the gel into human skin is tested as in Example 1. The concentration of the phthalimide in the epidermis is 2.28µg per cm² and the concentration of the phthalimide in the dermis is 2.83µg per cm² (average of 7 samples in each case).

### Example 4

Example 1 is repeated using 0.02 part of 4,5-bis(4-fluoroanilino)phthalimide instead of the phthalimide used in Example 1 and using 1 part of hydroxypropyl methylcellulose instead of the amount used in Example 1, to obtain a gel suitable for topical application in the treatment of psoriasis. The uptake of the phthalimide from the gel into human skin is tested as in Example 1, except that the skin samples are mounted in the diffusion cells for 16 hours and the receptor solution contains 0.1% bovine serum albumin in an aqueous medium buffered to pH7.4 with HEPES (4-(2-hydroxyethyl-1-piperazineethanesulphonic acid) instead of the solution used in Example 1. The concentration of the phthalimide in the epidermis and dermis respectively is 16µg per cm² and 10µg per cm².

### Example 5

Example 4 is repeated using 1.5 part of hydroxypropyl methylcellulose instead of the amount used in that Example to obtain a gel suitable for topical application in the treatment of psoriasis. The uptake of the phthalimide from the gel into human skin is measured as in Example 4. The concentration of the phthalimide in the epidermis and dermis respectively is 17µg per cm² and 13µg per cm².

### Example 6

Example 4 is repeated using 2.0 part of hydroxypropyl methylcellulose instead of the amount used in that Example to obtain a gel suitable for topical application in the treatment of psoriasis. The uptake of the phthalimide from the gel into human skin is measured as in Example 4. The concentration of the phthalimide in the epidermis and dermis respectively is 15µg per cm² and 5µg per cm².

### Example 7

Example 4 is repeated using 0.05 part of 4,5-bis(4-fluoroanilino)phthalimide instead of the amount used in Example 4, to obtain a gel suitable for topical application in the treatment of psoriasis. The uptake of the phthalimide from the gel into human skin is measured as in Example 4. The concentration of the phthalimide in the epidermis and dermis respectively is 13.3µg per cm² and 8.9µg per cm².

### Example 8

A solution containing 0.03 part 4,5-bis (anilino)phthalimide and 0.02 part butylated hydroxyanisole in 50 parts diethyleneglycol monoethyl ether is added to 50 parts of purified water at ambient temperature (20 to 30°C). Hydroxypropyl cellulose (2 parts) is added to the mixture whilst stirring vigorously. Stirring under vacuum is maintained during gelation of the mixture, which takes between 20 and 30 minutes at ambient temperature. The gel obtained is suitable for topical application in the treatment of psoriasis.

### Example 9

Example 8 is repeated using 0.02 part of 4,5-bis(anilino)phthalimide and 1.5 parts of hydroxypropyl cellulose instead of the amounts used in that Example. The gel obtained is suitable for topical application in the treatment of psoriasis.

### Example 10

Example 8 is repeated using 0.04 part of 4,5-bis(anilino)phthalimide instead of the amount used in that Example. The gel obtained is suitable for topical application in the treatment of psoriasis.

### Example 11

To a mixture of a polyethylene glycol having a molecular weight of 400 (480 parts) and diethylene glycol monoethyl ether (120 parts) is added, with stirring, 4,5-bis(anilino)phthalimide (0.06 part) and butylated hydroxyanisole (0.12 part). To the resulting solution hydroxypropyl cellulose (12 parts) is added and the mixture is heated to 80°C, with stirring, for 10 minutes before cooling to ambient temperature, when a gel suitable for topical application in the treatment of psoriasis is obtained.

### Example 12

Example 11 is repeated, using 0.12 part of 4,5-bis(anilino)phthalimide in place of the amount used in that Example, to give a gel suitable for topical application in the treatment of psoriasis.

### Example 13

Example 11 is repeated, using 0.24 part of 4,5-bis(anilino)phthalimide in place of the amount used in that Example, to give a gel suitable for topical application in the treatment of psoriasis.

### Example 14

Example 11 is repeated, Using 0.6 part of 4,5-bis(anilino)phthalimide instead of the amount used in that Example, to give a gel suitable for topical application in the treatment of psoriasis.

### Example 15

Water (33.38 parts) is heated to 75-85°C. Hydroxypropyl methylcellulose (1.5 parts) is added slowly to the heated water while stirring vigorously. The mixture is cooled slowly to 25°C under vacuum. To the cooled mixture is added a solution of 4,5-bis(anilino)phthalimide (0.1 part) and butylated hydroxyanisole (0.02part) in diethyleneglycol monoethyl ether. The resulting mixture is stirred at 25°C for 3 hours to give a gel suitable for topical application in treating psoriasis.

### Example 16

Example 15 is repeated, using 39.44 parts water, 0.04 part 4,5-bis(anilino)phthalimide and 59.0 parts diethylene glycol monoethyl ether instead of the amounts used in that Example, to give a gel suitable for topical application in the treatment of psoriasis.

## Claims

1. A composition suitable for topical administration in treatment of a hyperproliferative skin disease which is a gel comprising (A) a compound of formula 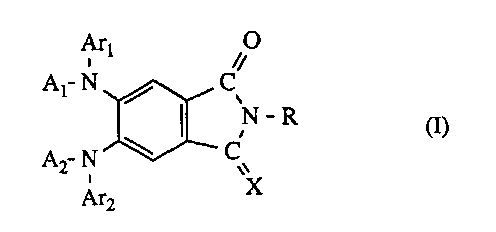 wherein A₁ and A₂ are each independently of the other hydrogen, lower alkyl, lower alkenyl, lower alkynyl, aryl, acyl, lower alkylsulfonyl or arylsulfonyl; or wherein A₁ and A₂ together form unsubstituted or lower alkyl- or hydroxy-substituted lower alkylene; Ar₁ and Ar₂ are each independently of the other aryl, heteroaryl or unsubstituted or substituted cycloalkyl; the group -C(=X)- is -C(=O)-, -C(=S)-, -CH₂- or -C(=CR₁R₂)- wherein R₁ and R₂ are each independently of the other hydrogen or lower alkyl; and R is hydrogen, lower alkyl, aryl-lower alkyl, aryl, amino, hydroxy or lower alkoxy; with the proviso that R is other than phenyl when A₁ and A₂ are hydrogen, Ar₁ and Ar₂ are phenyl and the group -C(=X)- is -C(=O)-; or a pharmaceutically acceptable salt thereof when a salt-forming group is present; (B) a C₁₋C₄ alkyl monoether of diethyleneglycol, (C) a gelling agent and (D) a solvent which is miscible with (B).

2. A composition according to claim 1 in which (A) is a compound of formula I wherein A₁ and A₂ are each hydrogen; Ar₁ and Ar₂ are each independently of the other unsubstituted phenyl or phenyl that is substituted by halogen, the group -C(=X)- is -C(=O)-, and R is hydrogen; or a pharmaceutically acceptable salt thereof.

3. A composition according to claim 1 in which (A) is 4,5-bis(anilino)phthalimide or 4,5-bis(4-fluoroanilino)phthalimide.

4. A composition according to any of the preceding claims, in which (B) is diethyleneglycol monoethyl ether.

5. A composition according to any of the preceding claims, in which the gelling agent (C) is a cellulosic polymer.

6. A composition according to claim 5, in which (C) is a cellulose ether.

7. A composition according to claim 6, in which the cellulose ether is hydroxypropyl cellulose or hydroxypropyl methylcellulose.

8. A composition according to any of the preceding claims, in which the solvent (D) is water, an organic solvent or a mixture of water with a water-miscible organic solvent.

9. A composition according to claim 8, in which the solvent (D) is water or a polyethylene glycol having a molecular weight up to 450.

10. A composition according to any of the preceding claims which contains (A) in an amount of 0.005 to 0.1% by weight.

11. A composition according to any of the preceding claims which contains (B) in an amount of 20 to 80% by weight.

12. A composition according to any of the preceding claims which contains (C) in an amount of 0.5 to 10% by weight.

13. A method of preparing a composition according to any of the preceding claims, which comprises either dissolving (A) in (B), mixing the resulting solution with the solvent (D) and adding the gelling agent (C) to the resulting mixture, or where (D) is organic, dissolving (A) in a mixture of (B) and (D) and adding the gelling agent (C) to the resulting solution.

14. A method of preparing a composition according to any of claims 1 to 12 which comprises mixing a solution of (A) in (B) with a gel-forming mixture of the gelling agent (C) and the solvent (D), the temperature of the mixture being controlled so that the viscosity of the mixture is such that (A) and (B) are dissolved therein before the gel is set.

15. Use of a composition according to any of claims 1 to 12 in the preparation of a medicament for treatment of hyperproliferative skin disease.

16. Use according to claim 15, in which the disease is psoriasis.
